# EUROPEAN PATENT SPECIFICATION

(11) **EP 2 805 157 B1**
(45) Date of publication and mention of the grant of the patent: **12.07.2017**
(21) Application number: 13704843.5
(22) Date of filing: 18.01.2013
(51) Int. Cl.: G01N 27/414

(54) **CHEMICAL SENSOR WITH CONDUCTIVE CUP-SHAPED SENSOR SURFACE**
CHEMISCHER SENSOR MIT LEITFÄHIGER BECHERFÖRMIGER SENSOROBERFLÄCHE
CAPTEUR CHIMIQUE À SURFACE DE CAPTEUR CONDUCTRICE EN FORME DE COUPELLE

(30) Priority: 19.01.2012 US 201213354108
(43) Date of publication of application: 26.11.2014
(73) Proprietor: Life Technologies Corporation, Carlsbad, CA 92008 (US); Li, Shifeng, Carlsbad, California 92008 (US); Bustillo, James, Carlsbad, California 92008 (US); Hinz, Wolfgang, Carlsbad, California 92008 (US)
(72) Inventor: LI, Shifeng, Carlsbad, California 92008 (US); BUSTILLO, James, Carlsbad, California 92008 (US); HINZ, Wolfgang, Carlsbad, California 92008 (US)
(74) Representative: Wöhler, Christian
(86) International application number: PCT/US2013/022129
(87) International publication number: WO 2013/109877

(56) References cited:
- EP-A2- 1 557 884
- US-A1- 2010 137 143
- US-A1- 2010 301 398
- US-A1- 2010 301 398
- CHIN Y-L ET AL: "TITANIUM NITRIDE MEMBRANE APPLICATION TO EXTENDED GATE FIELD EFFECT TRANSISTOR PH SENSOR USING VLSI TECHNOLOGY", JAPANESE JOURNAL OF APPLIED PHYSICS, JAPAN SOCIETY OF APPLIED PHYSICS, JP, vol. 40, no. 11, PART 01, 1 November 2001 (2001-11-01), pages 6311-6315, XP001089373, ISSN: 0021-4922, DOI: 10.1143/JJAP.40.6311
- YAN LIU ET AL: "An Extended CMOS ISFET Model Incorporating the Physical Design Geometry and the Effects on Performance and Offset Variation", IEEE TRANSACTIONS ON ELECTRON DEVICES, IEEE SERVICE CENTER, PISACATAWAY, NJ, US, vol. 58, no. 12, 1 December 2011 (2011-12-01), pages 4414-4422, XP011369500, ISSN: 0018-9383, DOI: 10.1109/TED.2011.2168821
- V.K. Khanna: "Ion-Sensitive Field-Effect Transistor (ISFET) - Based Chemical Sensors" In: "Sensor Technology Series : Chemical Sensors Comprehensive Sensor Technologies : Volume 5 Electrochemical and Optical Sensors", 1 July 2011 (2011-07-01), Momentum Press, XP055271257, ISBN: 978-1-60650-238-9 pages 171-274,

## Description

### FIELD OF THE DISCLOSURE

This disclosure, in general, relates to sensor arrays and methods for making same.

### BACKGROUND

Electronic sensor arrays are finding increased use for detecting analytes in fluids, such as gases or liquids. In particular, arrays of sensors based on field effect transistors are finding use in detecting ionic components, such as various cations, anions or pH. Such sensors are often referred to as ion-sensitive field effect transistors or ISFETs.

European patent application with publication number EP 1557884 discloses a sensor chip with an ISFET integrated on a semiconductor circuit device. The ISFET has a gate electrode that is either covered by an ion selection film or by a metal layer.

An article titled "Titanium Nitride Membrane Application to Extended Get Field Effect Transistor pH Sensor Using VLSI Technology" in Japanese Journal of Applied Physics, vol. 40 (2001), no. 11, pages 6311 to 6315, discloses a chemical sensor with an extended gate field effect transistor (EGFET) that is produced using a standard CMOS process. The EGFET has an aluminum layer electrode that is covered by a sensing layer made of titanium nitride.

The article "An Extended CMOS ISFET Model Incorporating the Physical Design Geometry and the Effects on Performance and Offset Variation" by Yan Liu et al. published in the IEEE transactions on electron devices (vol. 58, no. 12, 1 December 2011, pages 4414-4422) describes the formation of intrinsic passivation layers on the top metal layer of a floating gate based ISFET structure.

The chapter with title "Ion-Sensitive Field-Effect Transistor (ISFET) - Based Chemical Sensors" by V.K. Khanna of the textbook "Sensor Technology Series : Chemical Sensors Comprehensive Sensor Technologies : Volume 5, Electrochemical and Optical Sensors" describes on pages 171-274 different ISFET/EGFET gate dielectric materials and their corresponding pH response in mV/pH.

Recently, sensor arrays have found use in sequencing polynucleotides. Nucleotide addition results in the release of ionic species that influence the pH in a local environment. Sensors of the sensor arrays are used to detect changes in pH in the local environment resulting from the nucleotide addition. However, the pH of the local environment can be influenced by the interaction of various materials with hydrogen ions, leading to lower accuracy and less sensitivity to changes caused by nucleotide addition.

As such, an improved sensor array would be desirable.

### SUMMARY

In one implementation, a chemical detection device according to claim 1 is described herein. The device includes a chemically-sensitive field effect transistor including a floating gate conductor having an upper surface. A dielectric material defines a cavity extending to the upper surface of the floating gate conductor. A conductive layer is on a sidewall of the cavity and electrically communicating with the floating gate conductor. An inner surface of the conductive layer defines a well for the sensor.

In another implementation, a method for manufacturing a chemical detection device according to claim 8 is described. The method includes forming a chemically-sensitive field effect transistor including a floating gate conductor having an upper surface. The method also includes forming a dielectric material defining a cavity extending to the upper surface of the floating gate conductor. The method also includes forming a conductive layer on a sidewall of the cavity and electrically communicating with the floating gate conductor, an inner surface of the conductive layer defining a well for the sensor

Particular aspects of one more implementations of the subject matter described in this specification are set forth in the drawings and the description below. Other features, aspects, and advantages of the subject matter will become apparent from the description, the drawings, and the claims.

### BRIEF DESCRIPTION OF THE DRAWINGS

The present disclosure may be better understood, and its numerous features and advantages made apparent to those skilled in the art by referencing the accompanying drawings.
FIG. 1 illustrates a cross-sectional view of a flow cell and a portion of a flow chamber coupled to a sensor array.
FIG. 2 illustrates an expanded view of a well and a chemical sensor.
FIG. 3 illustrates a cross-sectional view of an upper portion of representative chemical sensors coupled to corresponding wells in accordance with an embodiment.
FIG. 4 illustrates a cross-sectional view of an upper portion of representative chemical sensors coupled to corresponding wells in accordance with another embodiment.
FIG. 5 illustrates a top view of a portion of representative chemical sensors coupled to corresponding wells.
FIG. 6 includes cross-sectional illustrations of exemplary well shapes.
FIG. 7 through FIG. 11 illustrate stages during exemplary manufacturing processes.
FIG. 12 illustrates a block diagram of components of a system for nucleic acid sequencing according to an exemplary embodiment.

The use of the same reference symbols in different drawings indicates similar or identical items.

### DETAILED DESCRIPTION

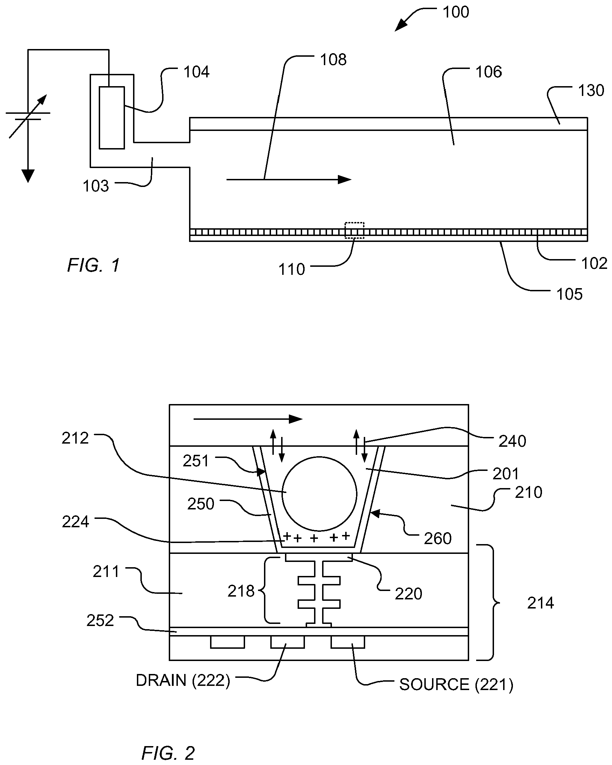

In a particular embodiment, a sequencing system includes a flow cell in which a sensor array having sensors as described herein is disposed, includes communication circuitry in electronic communication with the sensor array, and includes containers and fluid controls in fluidic communication with the flow cell. FIG. 1 illustrates a cross-sectional view of a flow cell 100 and a portion of a flow chamber 106 coupled to a sensor array 105 including chemical sensors as described herein. A reagent flow 108 flows across a surface of a microwell array 102, in which the reagent flow 108 flows over the open ends of wells (also referred to herein as microwells) of the microwell array 102. The microwell array 102 and a sensor array 105 together may form an integrated unit forming a lower wall (or floor) of flow cell 100. A reference electrode 104 may be fluidly coupled to flow chamber 106. Further, a flow cell cover 130 encapsulates flow chamber 106 to contain reagent flow 108 within a confined region.

FIG. 2 illustrates an expanded view of a representative well 201 and a corresponding chemical sensor 214, as illustrated at 110 of FIG. 1. The volume, shape, aspect ratio (such as base width-to-well depth ratio), and other dimensional characteristics of the wells may be selected based on the nature of the reaction taking place, as well as the reagents, byproducts, or labeling techniques (if any) that are employed. In the illustrated example, the chemical sensor 214 is a chemically-sensitive field effect transistor (chemFET), more specifically an ion-sensitive field effect transistor (ISFET) in this example. The chemical sensor 214 includes a floating gate structure 218 having a sensor plate 220 coupled to the well 201 via an electrically conductive layer 250 within the well 201. As can be seen in FIG. 2, the sensor plate 220 is the uppermost patterned conductive material in the floating gate structure 218. In the illustrated example, the floating gate structure 218 includes multiple patterned layers of conductive material within layers of dielectric material 211.

The chemical sensor 214 includes a source region 221 and a drain region 222 within a semiconductor substrate. The source region 221 and the drain region 222 comprise doped semiconductor material have a conductivity type different from the conductivity type of the substrate. For example, the source region 221 and the drain region 222 may comprise doped P-type semiconductor material, and the substrate may comprise doped N-type semiconductor material.

A channel region separates the source region 221 and the drain region 222. The floating gate structure 218 overlies the channel region, and is separated from the substrate 354 by a gate dielectric 252. The gate dielectric 252 may be for example silicon dioxide. Alternatively, other dielectrics may be used for the gate dielectric 252.

As shown in FIG. 2, the well 201 is within a cavity (or opening) extending through dielectric material 210 to the sensor plate 220. The cavity includes a sidewall 260 on which the conductive layer 250 is formed. In the illustrated embodiment, the conductive layer 250 is a conformal layer of electrically conductive material within the cavity, such that the conductive layer 250 is on the upper surface of the sensor plate 220. As a result of this structure, the inner surface 251 of the conductive layer 250 defines the well 201. That is, there is no intervening deposited material layer between the inner surface 251 of the conductive layer 250 and the corresponding well 201 for the sensor 214. As a result of this structure, the inner surface 251 of the conductive layer 250 is cup-shaped and acts as the sensing surface for the sensor 251.

The use of an electrically conductive material for the cup-shaped sensing surface, such as that provided in embodiments in which there is no intervening deposited layer between the conductive layer 250 and the well 201, can reduce the thermal resistance of the well 201 when containing solution during use, which in turn can reduce the overall noise of the device. During manufacturing and/or operation of the device, a thin oxide of the material of the conductive layer 250 may be grown which acts as a sensing material (e.g. an ion-sensitive sensing material) for the sensor 214, depending on the conductive material, the manufacturing processes performed and the conditions under which the device is operated. In an embodiment according to claim 1 the conductive layer 250 may be titanium nitride, and titanium oxide or titanium oxynitride may be grown on the inner surface 251 of the conductive layer 250 during manufacturing and/or during exposure to solutions during use. In other examples, not forming part of the claimed subject-matter, the conductive layer 250 may comprise other electrically conductive materials, such as metals or ceramics. The conductive material can be for example a metallic material or alloy thereof, or can be a ceramic material, or a combination thereof. An exemplary metallic material includes one of aluminum, copper, nickel, titanium, silver, gold, platinum, hafnium, tantalum, tungsten, or a combination thereof. In particular, the metal can include copper. In another example, the ceramic material can include one of titanium nitride, titanium aluminum nitride, titanium oxynitride, or a combination thereof.

In some examples, an additional sensing material (not shown) is deposited on the conductive layer 250. The sensing material may comprise one or more of a variety of different materials to facilitate sensitivity to particular ions. For example, silicon nitride or silicon oxynitride, as well as metal oxides such as silicon oxide, aluminum or tantalum oxides, generally provide sensitivity to hydrogen ions, whereas sensing materials comprising polyvinyl chloride containing valinomycin provide sensitivity to potassium ions. Materials sensitive to other ions such as sodium, silver, iron, bromine, iodine, calcium, and nitrate may also be used, depending upon the implementation.

In operation, reactants, wash solutions, and other reagents may move in and out of the well 201 by a diffusion mechanism 240. The chemical sensor 214 is responsive to (and generates an output signal related to) the amount of a charge 224 in the well 201 proximate to the conductive layer 250. Changes in the charge 224 cause changes in the voltage on the floating gate structure 218, which in turn changes in the threshold voltage of the transistor. This change in threshold voltage can be measured by measuring the current in the channel region between the source region 221 and a drain region 222. As a result, the chemical sensor 214 can be used directly to provide a current-based output signal on an array line connected to the source region 221 or drain region 222, or indirectly with additional circuitry to provide a voltage-based output signal.

In an example, reactions carried out in the well 201 can be analytical reactions to identify or determine characteristics or properties of an analyte of interest. Such reactions can generate directly or indirectly byproducts that affect the amount of charge proximate to the conductive layer 250. If such byproducts are produced in small amounts or rapidly decay or react with other constituents, multiple copies of the same analyte may be analyzed in the well 201 at the same time in order to increase the output signal generated. In an example, multiple copies of an analyte may be attached to a solid phase support 212, either before or after deposition into the well 201. The solid phase support 212 may be microparticles, nanoparticles, beads, solid or porous comprising gels, or the like. For simplicity and ease of explanation, solid phase support 212 is also referred herein as a particle. For a nucleic acid analyte, multiple, connected copies may be made by rolling circle amplification (RCA), exponential RCA, or like techniques, to produce an amplicon without the need of a solid support.

In various examples, the methods, systems, and computer readable media described herein may advantageously be used to process and/or analyze data and signals obtained from electronic or charged-based nucleic acid sequencing. In electronic or charged-based sequencing (such as, pH-based sequencing), a nucleotide incorporation event may be determined by detecting ions (e.g., hydrogen ions) that are generated as natural by-products of polymerase-catalyzed nucleotide extension reactions. This may be used to sequence a sample or template nucleic acid, which may be a fragment of a nucleic acid sequence of interest, for example, and which may be directly or indirectly attached as a clonal population to a solid support, such as a particle, microparticle, bead, etc. The sample or template nucleic acid may be operably associated to a primer and polymerase and may be subjected to repeated cycles or "flows" of deoxynucleoside triphosphate ("dNTP") addition (which may be referred to herein as "nucleotide flows" from which nucleotide incorporations may result) and washing. The primer may be annealed to the sample or template so that the primer's 3' end can be extended by a polymerase whenever dNTPs complementary to the next base in the template are added. Then, based on the known sequence of nucleotide flows and on measured output signals of the chemical sensors indicative of ion concentration during each nucleotide flow, the identity of the type, sequence and number of nucleotide(s) associated with a sample nucleic acid present in a well coupled to a chemical sensor can be determined.

The values of the output signals of the chemical sensors indicate physical and/or chemical parameters of one or more reactions taking place in the corresponding reaction regions in the microwell array. For example, in an example, the values of the output signals may be processed using the techniques disclosed in Rearick et al., U.S. Pat. Appl. No. 13/339,846, filed December 29, 2011, based on U.S. Prov. Pat. Appl. Nos. 61/428,743, filed December 30, 2010, and 61/429,328, filed January 3, 2011, and in Hubbell, U.S. Pat. Appl. No. 13/339,753, filed December 29, 2011, based on U.S. Prov. Pat. Appl. No 61/428,097, filed December 29, 2010.

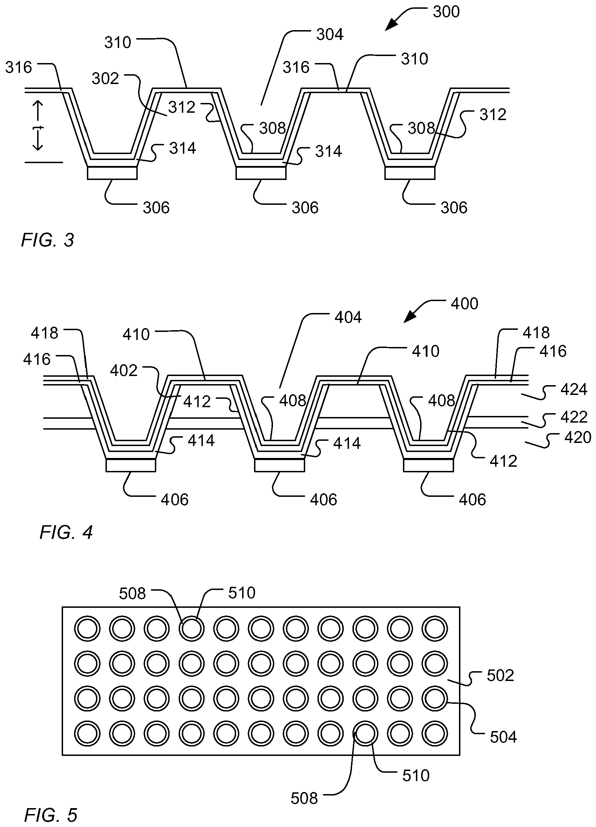

FIG. 3 illustrates a cross-sectional view of an upper portion of representative chemical sensors coupled to corresponding wells. In the illustrated example, three wells and upper portions of chemical sensors are shown, representing a small portion of an array that can include millions of wells and sensors.

As shown in FIG. 3, each well 304 overlies the sensor plate 306 of a corresponding chemical sensor. In the illustrated example, the sensor plate 306 is the uppermost patterned conductive material in the floating gate structure in transistors of the chemical sensors. The sensor plate 306 is coupled to the channel of the transistor (not shown) of the chemical sensor via multiple patterned layers of conductive material (now shown), similar to the discussion above with respect to FIG. 2.

The wells 304 are within respective cavities extending through dielectric material 302. Each cavity includes a sidewall 312 on which conductive layer 314 is formed. In the illustrated embodiment, the conductive layer 314 is a conformal layer of material within the cavity, such that the conductive layer 314 is on the upper surface of the corresponding sensor plate 306.

The dielectric material 302 also includes an upper surface 310 extending between adjacent wells 304. As can be seen in FIG. 3, the conductive material 314 does not extend along the upper surface 310 between adjacent wells, so that the sensor plates 306 and underlying circuitry of adjacent sensors are electrically isolated from one another.

The dielectric material 302 can be formed of one or more layers of material. In an example, the dielectric 302 can have a thickness (t) extending from the upper surface of the sensor plate 306 to the upper surface 310 in a range of 0.3 micrometers to 10 micrometers, such as a range of 0.5 micrometers to 6 micrometers. In some embodiments, the wells 304 can have a characteristic diameter, defined as the square root of 4 times the cross-sectional area (A) divided by Pi (e.g., sqrt(4*A/π), of not greater than 5 micrometers, such as not greater than 3.5 micrometers, not greater than 2.0 micrometers, not greater than 1.6 micrometers, not greater than 1.0 micrometers, not greater than 0.8 micrometers or even not greater than 0.6 micrometers.

In the illustrated embodiment, the conductive layer 314 extends up along the entire sidewall 312 of the well to the upper surface 310 of the dielectric material 302. In other embodiments, the conductive layer 314 may only extend from the sensor plate up along a portion of the sidewall 312. For example, the conductive layer 314 can extend at least 40% along the sidewall 312, such as at least 50%, at least 65%, at least 75%, or even at least 85% along the sidewall 312.

The conductive layer 314 comprises one or more layers of electrically conductive material. For example, the conductive material can have a volume resistivity of not greater than 6.0x10⁷ ohm-m at 25°C. In particular, the volume resistivity can be not greater than 1.0x10⁷ ohm-m at 25°C, such as not greater than 5.0x10⁶ ohm-m, or not greater than 2.0x10⁶ ohm-m at 25°C. According to the independent claims, the conductive material is a ceramic material. Another example, not falling under the scope of the independent claims, is a metallic material including aluminum, copper, nickel, titanium, silver, gold, platinum, or a combination thereof. In particular, the metal can include copper. According to the independent claims, the ceramic material can include titanium nitride.

According to the independent claims, a sensing material (also referred to herein as a passivation structure) 316 can be disposed over the conductive layer 314 and optionally the upper surface 310 of the dielectric material 302. In particular, the passivation structure 316 can have a high intrinsic buffer capacity. For example, the passivation structure 316 can have an intrinsic buffer capacity of at least 2x10¹⁷ groups/m². Intrinsic buffer capacity is defined as the surface density of hydroxyl groups on a surface of a material measured at a pH of 7. For example, the passivation structure 316 can have an intrinsic buffer capacity of at least 4x10¹⁷ groups/m², such as at least 8x10¹⁷ groups/m², at least 1x10¹⁸ groups/m², or even at least 2x10¹⁸ groups/m². In an example, the passivation structure 316 has an intrinsic buffer capacity of not greater than 1x10²¹ groups/m².

In particular, the passivation structure 316 can include an inorganic material. Such material can include an oxide of titanium or titanium nitride. In an example not forming part of the claimed subject-matter, the material can include an oxide of tantalum. In another example, the material includes an oxide of zirconium. In a further example, the upper surface 310 can be coated with a pH buffering coating. An exemplary pH buffering coating can include a functional group, such as phosphate, phosphonate, catechol, nitrocatechol, boronate, phenylboronate, imidazole, silanol, another pH-sensing group, or a combination thereof.

In an example, the passivation structure 316 can have a thickness in a range of 5 nm to 100 nm, such as a range of 10 nm to 70 nm, a range of 15 nm to 65 nm, or even a range of 20 nm to 50 nm.

While FIG. 3 illustrates a single-layer dielectric material 302, a single-layer conductive layer 314 and a single-layer passivation structure 316, the system can include, one or more dielectric layers, one or more conductive layers, and/or one or more passivation layers. For example, as illustrated in FIG. 4, a dielectric material 402 defines cavities containing wells 404 positioned over sensor plates 406. The dielectric material 402 can be formed of one or more layers 420, 422, or 424. In the example illustrated in FIG. 4, a layer 420 of dielectric material 402 can include an oxide of silicon or TEOS. A layer 422 can include a nitride of silicon, and a layer 424 can include an oxide of silicon or TEOS.

The dielectric material 402 defines cavities containing wells 404 having a lower surface 408 defined by a passivation layer 418. Each cavity includes a sidewall 412 extending between the upper surface of the corresponding sensor plate 406 and the upper surface 410 of the dielectric material 402. A conductive layer 414 in contact with the sensor plate 406 can extend across the upper surface of the sensor plate 406 and at least partially along the sidewall 412 of the dielectric material 402. For example, the conductive layer 414 can extend at least 40% along the sidewall 412, such as at least 50%, at least 65%, at least 75%, or even at least 85% along the sidewall 412. While illustrated as a single layer, the conductive layer 414 can include one or more layers, such as one or more metal layers or one or more ceramic layers.

The conductive layer 414 comprises one more conductive materials. For example, the conductive material can have a volume resistivity of not greater than 6.0x10⁷ ohm-m at 25°C. In particular, the volume resistivity can be not greater than 1.0x10⁷ ohm-m at 25°C, such as not greater than 5.0x10⁶ ohm-m, or not greater than 2.0x10⁶ ohm-m at 25°C. According to the independent claims, the conductive material is a ceramic material. Another example, not falling under the scope of the independent claims, is a metallic material including aluminum, copper, nickel, titanium, silver, gold, platinum, or a combination thereof. In particular, the metal can include copper. According to the independent claims, the ceramic material can include titanium nitride.

Optionally, one or more passivation layers (also referred to herein as sensing material) 416 or 418 can be disposed over the dielectric material 402 and conductive layer 414. In the illustrated example, the passivation layers 416 or 418 are disposed over the dielectric material 402 and conductive layer 414, including over the upper surface 410 of the well wall structure 402. In an example, the passivation layer 416 can include aluminum oxide and the passivation layer 418 can include tantalum oxide. Alternatively, one or more additional layers formed of one or more additional materials, such as aluminum oxide, tantalum oxide, or zirconium oxide, can be formed as part of a passivation structure over the conductive structure 414 and the well wall structure 402. In a particular example, the passivation layer 418 defining an outer surface has an intrinsic buffer capacity of at least 2.0x10¹⁷ groups/m². Intrinsic buffer capacity is defined as the surface density of hydroxyl groups on the surface of material measure at a pH of 7. For example, the passivation layer 418 can have an intrinsic buffer capacity of at least 4x10¹⁷ groups/m², such as at least 8x10¹⁷ groups/m², at least 1x10¹⁸ groups/m², or even at least 2x10¹⁸ groups/m². In an example, the passivation layer 418 has an intrinsic buffer capacity of not greater than 1x10²¹ groups/m².

In a further example, the passivation layer 418 can be coated with a pH buffering coating. An exemplary pH buffering coating can include a functional group, such as phosphate, phosphonate, catechol, nitrocatechol, boronate, phenylboronate, imidazole, silanol, another pH-sensing group, or a combination thereof.

FIG. 5 illustrates a top view of a portion of representative chemical sensors coupled to corresponding wells. In the illustrated embodiment, the cavities defined by the dielectric material 502, and thus the conductive layer 508 on the sidewall within each well 504, have circular cross-sections. Alternatively, these may be non-circular. For example, the cross-sections may be hexagonal.

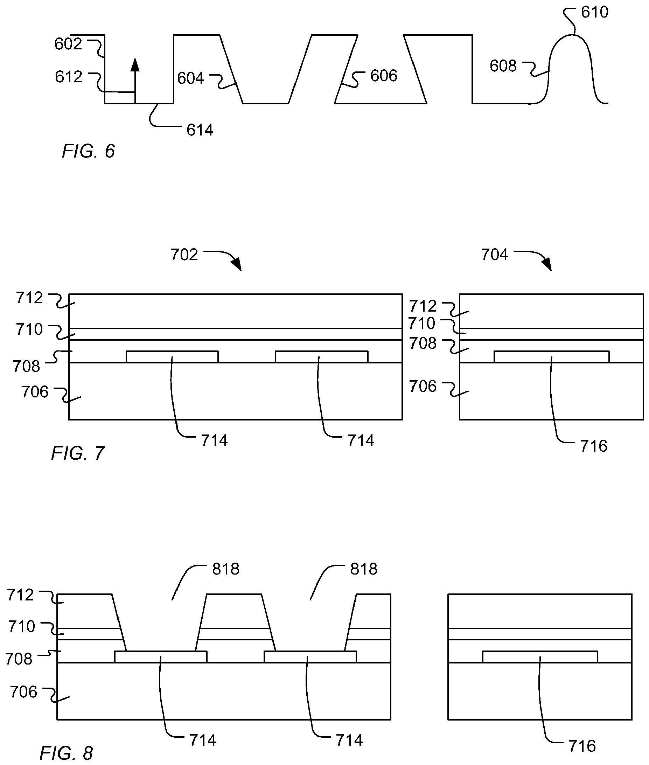

While the sidewall 312 of FIG. 3 and sidewall 412 of FIG. 4 of the wells are illustrated as extending substantially vertically and outwardly, the sidewall surface can extend in various directions and have various shapes, depending on the techniques used to form them. Substantially vertically denotes extending in a direction having a component that is normal to the upper surface of the sensor plate. For example, as illustrated in FIG. 6, a sidewall 602 can extend vertically, being parallel to a normal component 612 of a surface 614 parallel to the top surface of the sensor plate. In another example, the well is tapered such that sidewall 604 extends in an outward direction away from the sensor pad, providing a larger opening at the top of the well than at the bottom of the well. In an alternative example, a sidewall 606 extends inward direction, providing an opening area at the top of the well that is smaller than an area at the bottom of the well. The sidewall 606 extends in a direction having a component parallel to the normal component 612 of the surface 614.

While the sidewalls 602, 604, or 606 are illustrated as straight lines, some semiconductor or CMOS manufacturing processes can result in structures having nonlinear shapes. In particular, sidewall, such as sidewall 608, and upper surfaces, such as upper surface 610, can be arcuate in shape or take various nonlinear forms. While the structures and devices illustrated herewith are depicted as having linear layers, surfaces, or shapes, actual layers, surfaces, or shapes resulting from semiconductor processing may differ to some degree, possibly including nonlinear and arcuate variations of the illustrated embodiment.

Such well structures as illustrated in FIG. 2, FIG. 3 or FIG. 4 can be formed by depositing a layer of conductive material over a structure including the dielectric material defining cavities overlying the sensor plates of floating gate structures of the transistors of the chemical sensors, planarizing or etching the conductive material, and optionally depositing a passivation material over the conductive material and the dielectric material. The structure including the transistors and the cavities in the dielectric material may for example be formed using the techniques described in U.S. Patent Application Publication No. 2010/0300559, No. 2010/0197507, No. 2010/0301398, No. 2010/0300895, No. 2010/0137143, and No. 2009/0026082, and U.S. Patent No. 7,575,865.

As illustrated in FIG. 7, a portion 702 of a device proximal to a sensor array is illustrated, and a portion 704 of the device proximal to electrical contacts is illustrated. One or more layers 708, 710 or 712 can be deposited over a CMOS formed structure 706, sensor plates 714 coupled to underlying circuitry (not shown), and contact pad 716. In an example, one or more layers of an oxide of silicon, a nitride of silicon, or TEOS can be deposited to overlie the sensor plate 714 and contact pad 716. In the illustrated example, a layer 708 of silicon oxide or TEOS is deposited over the CMOS structure 706. A layer 710 of silicon nitride is deposited over the layer 708, and a layer 712 of silicon oxide or TEOS is deposited over the layer 710. The total thickness of the one or more layers 708, 710 or 712 can be in a range of 0.3 µm to 10 µm, such as a range of 0.5 µm to 6 µm.

As illustrated in FIG. 8, the layers 708, 710, or 712 can be etched down to the sensor plate to form a cavity 818 defined by the remainder of the layer 708, 710, or 712. In an example, the cavity 818 can be formed using a wet etch, a plasma etch, or combination thereof. In particular, a fluorinated plasma etch process can be utilized to expose the sensor plates 714. As illustrated in FIG. 8, the layers can be masked to prevent exposure of the conductive pads 716.

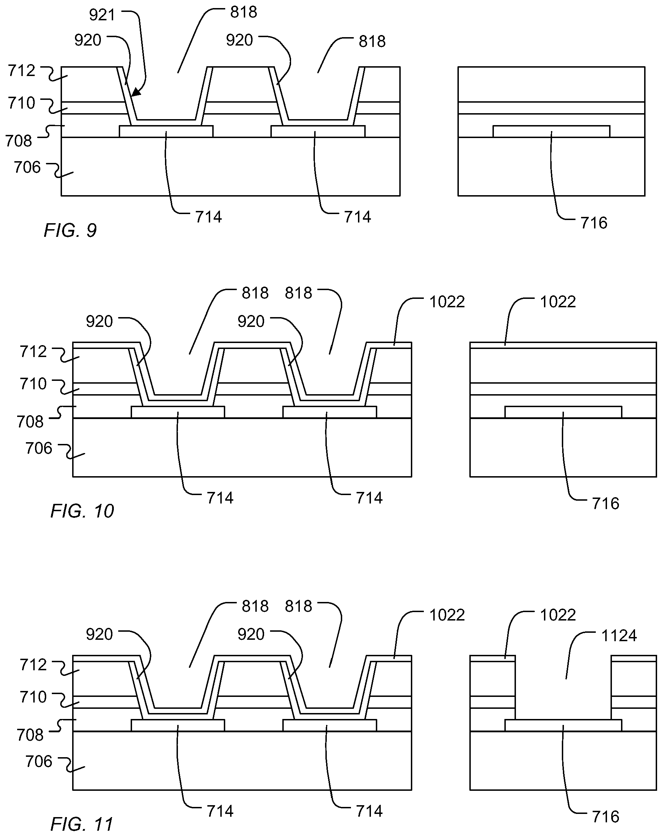

As illustrated in FIG. 9, a conductive layer 920 can be conformally deposited over a lower surface of the cavity 818 and along at least a portion of the sidewall of the cavity 818 defined by the layers 708, 710, or 712. For example, the conductive layer 920 can be deposited using sputtering, atomic layer deposition, or a liquid deposition technique. The conductive layer 920 can then be planarized to remove the conductive material from an upper surface of the well wall structure defined by the layers 708, 710, or 712. For example, a chemical mechanical process (CMP) may be performed.

In an example, the conductive layer 920 can be formed of a metal, a ceramic, or combination thereof. An exemplary metal includes aluminum, copper, nickel, titanium, silver, gold, platinum, or a combination thereof. In particular, the metal can include copper. In another example, the ceramic material can include titanium nitride, titanium aluminum nitride, titanium oxynitride, or a combination thereof. In particular, the titanium oxynitride is a high nitrogen content titanium oxynitride. Further, the titanium aluminum nitride can be a low aluminum content titanium aluminum nitride. The conductive material can have a volume resistivity of not greater than 6.0x10⁷ ohm-m at 25°C. In particular, the volume resistivity can be not greater than 1.0x10⁷ ohm-m at 25°C, such as not greater than 5.0x10⁶ ohm-m, or not greater than 2.0x10⁶ ohm-m at 25°C.

Optionally, one or more passivation layers can be deposited over the conductive layer 920. For example, as illustrated in FIG. 10, a passivation layer 1022 can be deposited over the conductive layer 920 and the well wall structure defined by layers 708, 710 or 712. An optional passivation layer 1022 can be formed of a ceramic material, such as an oxide of aluminum, hafnium, tantalum, zirconium, or any combination thereof. In particular, the passivation layer 1022 can include aluminum oxide, tantalum oxide, or combination thereof. In a particular example, the passivation layer 1022 can include zirconium oxide. In an example, the passivation layer has a high intrinsic buffer capacity such as an intrinsic buffer capacity of at least 2.0x10¹⁷ groups/m². For example, the passivation layer 1022 can have an intrinsic buffer capacity of at least 4x10¹⁷ groups/m², such as at least 8x10¹⁷ groups/m², at least 1x10¹⁸ groups/m², or even at least 2x10¹⁸ groups/m². In an example, the passivation layer 1022 has an intrinsic buffer capacity of not greater than 1x10²¹ groups/m².

Following formation of the conductive layer 920 and optional passivation layer 1022, the contact pad 716 can be exposed. For example, the wells 818 and dielectric material in proximity to the sensor pads 714 can be masked and an opening 1124 can be formed to expose the contact pad 716. For example, the contact pad 716 can be exposed using a wet etch, a plasma etch, or combination thereof. In particular, a fluorinated plasma etch process can be utilized to form access 1124 that terminates at the contact pad 716. In a particular example, the access 1124 can be filled with a conductive material to provide an electrical connection to the contact pad 716.

Referring back to FIG 9, as described above, in some implementations a passivation layer is not deposited over the conductive layer 920. In such a case, process step illustrated in FIG. 10 is omitted, and the inner surface 921 of the conductive layer 920 for each sensor defines the well for that sensor. In addition, the portion of the conductive layer 920 on the upper surface of the sensor plate 714 defines a bottom of the well. That is, in the completed device, there is no intervening deposited material layer between the conductive layer 920 and the corresponding well for that sensor. As a result of this structure, the inner surface of the conductive layer 920 acts as the sensing surface for that sensor.

The use of an electrically conductive material for the sensing surface, such as that provided in examples in which a passivation layer is not deposited over the conductive layer, can reduce the thermal resistance of the well when containing solution, which in turn can reduce the overall noise during operation. During manufacturing and/or operation of the device, a thin oxide of the material of the conductive layer 920 may be grown which acts as an ion-sensitive sensing material for the sensor, depending on the material, the manufacturing process steps and the conditions under which the device is operated. For example, in one embodiment the conductive layer 920 may be titanium nitride, and titanium oxide or titanium oxynitride may be grown on the inner surface of the conductive layer 920.

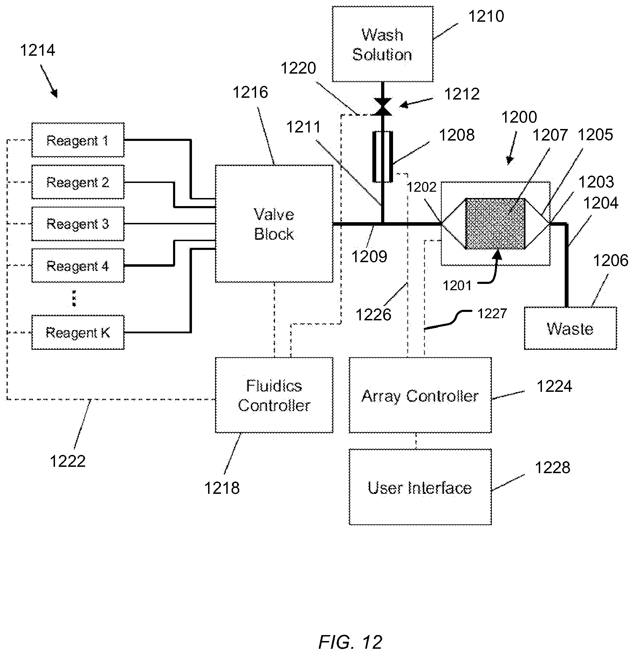

FIG. 12 illustrates a block diagram of components of a system for nucleic acid sequencing according to an exemplary embodiment. The components include a flow cell 1201 on an integrated circuit device 1200, a reference electrode 1208, a plurality of reagents 1214 for sequencing, a valve block 1216, a wash solution 1210, a valve 1212, a fluidics controller 1218, lines 1220/1222/1226, passages 1204/1209/1211, a waste container 1206, an array controller 1224, and a user interface 1228. The integrated circuit device 1020 includes a microwell array 1207 overlying a sensor array that includes chemical sensors as described herein. The flow cell 1201 includes an inlet 1202, an outlet 1203, and a flow chamber 1205 defining a flow path of reagents over the microwell array 1207.

The reference electrode 1208 may be of any suitable type or shape, including a concentric cylinder with a fluid passage or a wire inserted into a lumen of passage 1211. The reagents 1214 may be driven through the fluid pathways, valves, and flow cell 1201 by pumps, gas pressure, or other suitable methods, and may be discarded into the waste container 1206 after exiting the outlet 1203 of the flow cell 1201. The fluidics controller 1218 may control driving forces for the reagents 1214 and the operation of valve 1212 and valve block 1216 with suitable software.

The microwell array 1207 includes an array of reaction regions as described herein, also referred to herein as microwells or wells, which are operationally associated with corresponding chemical sensors in the sensor array. For example, each reaction region may be coupled to a chemical sensor suitable for detecting an analyte or reaction property of interest within that reaction region. The microwell array 1207 may be integrated in the integrated circuit device 1200, so that the microwell array 1207 and the sensor array are part of a single device or chip.

The flow cell 1201 may have a variety of configurations for controlling the path and flow rate of reagents 1214 over the microwell array 1207. The array controller 1224 provides bias voltages and timing and control signals to the integrated circuit device 1200 for reading the chemical sensors of the sensor array. The array controller 1224 also provides a reference bias voltage to the reference electrode 1208 to bias the reagents 1214 flowing over the microwell array 1207.

During an experiment, the array controller 1224 collects and processes output signals from the chemical sensors of the sensor array through output ports on the integrated circuit device 1200 via bus 1227. The array controller 1224 may be a computer or other computing means. The array controller 1224 may include memory for storage of data and software applications, a processor for accessing data and executing applications, and components that facilitate communication with the various components of the system in FIG. 12.

The user interface 1228 may display information about the flow cell 1201 and the output signals received from chemical sensors in the sensor array on the integrated circuit device 1200. The user interface 1228 may also display instrument settings and controls, and allow a user to enter or set instrument settings and controls.

In an example, during the experiment the fluidics controller 1218 may control delivery of the individual reagents 1214 to the flow cell 1201 and integrated circuit device 1200 in a predetermined sequence, for predetermined durations, at predetermined flow rates. The array controller 1224 can then collect and analyze the output signals of the chemical sensors indicating chemical reactions occurring in response to the delivery of the reagents 1214.

During the experiment, the system may also monitor and control the temperature of the integrated circuit device 1200, so that reactions take place and measurements are made at a known predetermined temperature.

The system may be configured to let a single fluid or reagent contact the reference electrode 1208 throughout an entire multi-step reaction during operation. The valve 1212 may be shut to prevent any wash solution 1210 from flowing into passage 1209 as the reagents 1214 are flowing. Although the flow of wash solution may be stopped, there may still be uninterrupted fluid and electrical communication between the reference electrode 1208, passage 1209, and the microwell array 1207. The distance between the reference electrode 1208 and the junction between passages 1209 and 1211 may be selected so that little or no amount of the reagents flowing in passage 1209 and possibly diffusing into passage 1211 reach the reference electrode 1208. In an exemplary embodiment, the wash solution 1210 may be selected as being in continuous contact with the reference electrode 108, which may be especially useful for multi-step reactions using frequent wash steps.

## Claims

1. A chemical detection device comprising:
a chemically-sensitive field effect transistor including a floating gate conductor having an upper surface;
a dielectric material defining a cavity extending to the upper surface of the floating gate conductor; and
a conductive layer on a sidewall of the cavity and electrically communicating with the floating gate conductor, an inner surface of the conductive layer defining a well for the sensor, **characterized in that**
the conductive layer is titanium nitride, and titanium oxide or titanium oxynitride is grown on the inner surface of the conductive layer.

2. The device of claim 1, wherein the conductive layer is on the upper surface of the floating gate conductor to define a bottom of the well.

3. The device of claim 1, wherein the conductive layer does not extend into a second well of an adjacent sensor.

4. The device of claim 1, wherein the conductive layer is formed of a material having a volume resistivity of not greater than 6.0x10⁷ ohm-m at 25°C or than 1.0x10⁷ ohm-m at 25°C.

5. The device of claim 1, wherein the conductive layer extends incompletely up the sidewall of the cavity.

6. The device of claim 5, wherein the conductive layer extends at least 40% up the sidewall of the cavity.

7. The device of claim 1, wherein the well has a diameter of not greater than 5 micrometers or than 0.6 micrometers.

8. A method for manufacturing a chemical detection device, the method comprising:
forming a chemically-sensitive field effect transistor including a floating gate conductor having an upper surface;
forming a dielectric material defining a cavity extending to the upper surface of the floating gate conductor;
forming a conductive layer on a sidewall of the cavity and electrically communicating with the floating gate conductor, an inner surface of the conductive layer defining a well for the sensor, **characterized in that**
the conductive layer is titanium nitride; and
growing titanium oxide or titanium oxynitride on the inner surface of the conductive layer.

9. The method of claim 8, wherein forming the conductive layer comprises:
depositing the conductive layer within the cavity and on an upper surface of the dielectric material; and
removing conductive material from the upper surface of the dielectric material.

10. The method of claim 8, wherein forming the conductive layer includes forming the conductive layer on the upper surface of the floating gate conductor to define a bottom of the well.

11. The method of claim 8, wherein the conductive layer is formed of a material having a volume resistivity of not greater than 6.0x10⁷ ohm-m at 25°C or than 1.0x10⁷ ohm-m at 25°C.

## Patentansprüche

1. Vorrichtung zum chemischen Nachweisen, umfassend:
einen chemisch sensitiven Feldeffekttransistor mit einem Floating-Gate-Leiter, der eine obere Oberfläche aufweist;
ein dielektrisches Material, das einen Hohlraum festlegt, der sich zu der oberen Oberfläche des Floating-Gate-Leiters erstreckt; und
eine leitfähige Schicht an einer Seitenwand des Hohlraums und elektrisch in Verbindung mit dem Floating-Gate-Leiter, wobei eine innere Oberfläche der leitfähigen Schicht eine Mulde für den Sensor festlegt,
**dadurch gekennzeichnet, dass** die leitfähige Schicht Titannitrid ist und Titanoxid oder Titanoxynitrid auf der inneren Oberfläche der leitfähigen Schicht aufgebracht ist.

2. Vorrichtung nach Anspruch 1, wobei sich die leitfähige Schicht auf der oberen Oberfläche des Floating-Gate-Leiters befindet, um einen Boden der Mulde festzulegen.

3. Vorrichtung nach Anspruch 1, wobei sich die leitfähige Schicht nicht bis in eine zweite Mulde eines angrenzenden Sensors erstreckt.

4. Vorrichtung nach Anspruch 1, wobei die leitfähige Schicht aus einem Material erzeugt ist, das einen Volumenwiderstand bei 25 °C von nicht größer als 6,0 x 10⁷ Ohmmeter oder als 1,0 x 10⁷ Ohmmeter bei 25 °C hat.

5. Vorrichtung nach Anspruch 1, wobei sich die leitfähige Schicht an der Seitenwand des Hohlraums nicht vollständig nach oben erstreckt.

6. Vorrichtung nach Anspruch 5, wobei sich die leitfähige Schicht zu mindestens 40% an der Seitenwand des Hohlraums nach oben erstreckt.

7. Vorrichtung nach Anspruch 1, wobei die Mulde einen Durchmesser von nicht mehr als 5 Mikrometer oder als 0,6 Mikrometer hat.

8. Verfahren zum Herstellen einer Vorrichtung zum chemischen Nachweisen, wobei das Verfahren umfasst:
Erzeugen eines chemisch sensitiven Feldeffekttransistors, einschliessend einen Floating-Gate-Leiter, der eine obere Oberfläche hat;
Erzeugen eines dielektrischen Materials, das einen Hohlraum festlegt, der sich zu der oberen Oberfläche des Floating-Gate-Leiters erstreckt;
Erzeugen einer leitfähigen Schicht an einer Seitenwand des Hohlraums und elektrisch in Verbindung mit dem Floating-Gate-Leiter, wobei eine innere Oberfläche der leitfähigen Schicht eine Mulde für den Sensor festlegt,
**dadurch gekennzeichnet, dass**
die leitfähige Schicht Titannitrid ist; und
Aufbringen von Titanoxid oder Titanoxynitrid auf der inneren Oberfläche der leitfähigen Schicht.

9. Verfahren nach Anspruch 8, wobei das Erzeugen der leitfähigen Schicht umfasst:
Abscheiden der leitfähigen Schicht innerhalb des Hohlraums und auf einer oberen Oberfläche des dielektrischen Materials; und
Entfernen des leitfähigen Materials von der oberen Oberfläche des dielektrischen Materials.

10. Verfahren nach Anspruch 8, wobei das Erzeugen der leitfähigen Schicht das Erzeugen der leitfähigen Schicht auf der oberen Oberfläche des Floating-Gate-Leiters einschließt, um einen Boden der Mulde festzulegen.

11. Verfahren nach Anspruch 8, wobei die leitfähige Schicht aus einem Material erzeugt wird, das einen Volumenwiderstand bei 25 °C von nicht größer als 6,0 x 10⁷ Ohmmeter oder als 1,0 x 10⁷ Ohmmeter bei 25 °C hat.

## Revendications

1. Dispositif de détection chimique comprenant :
un canal transistor à effet de champ et chimiquement sensible comprenant un conducteur de grille flottante présentant une surface supérieure ;
un matériau diélectrique définissant une cavité s'étendant jusqu'à la surface supérieure du conducteur de la grille flottante ; et
une couche conductrice située sur la paroi latérale de la cavité et communiquant électriquement avec le conducteur de grille flottante, une surface intérieure de la couche conductrice définissant un puits pour le capteur, **caractérisé en ce que**
la couche conductrice est du nitrure de titane, et l'oxyde de titane ou l'oxynitrure de titane est développé sur la surface intérieure de la couche conductrice.

2. Dispositif selon la revendication 1, dans lequel la couche conductrice est sur la surface supérieure du conducteur de grille flottante pour définir un fond du puits.

3. Dispositif selon la revendication 1, dans lequel la couche conductrice ne s'étend pas dans un deuxième puits d'un capteur adjacent.

4. Dispositif selon la revendication 1, dans lequel la couche conductrice est constituée d'un matériau présentant une résistivité de volume inférieure à 6,0.10⁷ Ω.m à 25 °C ou à 1,0.10⁷ Ω.m à 25 °C.

5. Dispositif selon la revendication 1, dans lequel la couche conductrice s'étend de manière incomplète vers le haut de la paroi latérale de la cavité.

6. Dispositif selon la revendication 5, dans lequel la couche conductrice s'étend au moins sur 40 % vers le haut de la paroi latérale de la cavité.

7. Dispositif selon la revendication 1, dans lequel le puits a un diamètre inférieur à 5 µm ou à 0,6 µm.

8. Procédé de fabrication d'un dispositif de détection chimique, le procédé comprenant :
la formation d'un transistor à effet de champ et chimiquement sensible comprenant un conducteur de grille flottante présentant une surface supérieure ;
la formation d'un matériau diélectrique définissant une cavité s'étendant jusqu'à la surface supérieure du conducteur de la grille flottante ;
la formation d'une couche conductrice sur une paroi latérale de la cavité et communiquant électriquement avec le conducteur de grille flottante, une surface intérieure de la couche conductrice définissant un puits pour le capteur, **caractérisé en ce que**
la couche conductrice est du nitrure de titane, et
le développement d'oxyde de titane ou d'oxynitrure de titane sur la surface intérieure de la couche conductrice.

9. Procédé selon la revendication 8, dans lequel la formation de la couche conductrice comprend :
le dépôt de la couche conductrice dans la cavité et sur une surface supérieure du matériau diélectrique ; et
l'élimination du matériau conducteur de la surface supérieure du matériau diélectrique.

10. Procédé selon la revendication 8, dans lequel la formation de la couche conductrice comprend la formation de la couche conductrice sur la surface supérieure du conducteur de grille flottante pour définir un fond du puits.

11. Procédé selon la revendication 8, dans lequel la couche conductrice est formée d'un matériau présentant une résistivité volumique inférieure à 6,0.10⁷ Ω.m à 25 °C ou à 1,0.10⁷ Ω.m à 25 °C.
